(19) Europäisches Patentamt
European Patent Office
Office européen des brevets

(11) Veröffentlichungsnummer: **0 019 785**
**B1**

(12) # EUROPÄISCHE PATENTSCHRIFT

(45) Veröffentlichungstag der Patentschrift: **25.07.84**

(21) Anmeldenummer: **80102613.9**

(22) Anmeldetag: **12.05.80**

(51) Int. Cl.³: **C 09 B 62/20,** D 06 P 3/66, D 06 P 3/10, C 07 D 239/30

(54) **Reaktivfarbstoffe, ihre Herstellung und Verwendung zum Färben und Bedrucken von Cellulose oder Polypeptiden.**

(30) Priorität: **23.05.79 DE 2920949**

(43) Veröffentlichungstag der Anmeldung:
**10.12.80 Patentblatt 80/25**

(45) Bekanntmachung des Hinweises auf die Patenterteilung:
**25.07.84 Patentblatt 84/30**

(84) Benannte Vertragsstaaten:
**CH DE FR GB LI**

(56) Entgegenhaltungen:
**FR - A - 2 360 640**
**LU - A - 59 203**
**US - A - 3 822 263**

(73) Patentinhaber: **BAYER AG**
**Konzernverwaltung RP Patentabteilung**
**D-5090 Leverkusen 1 Bayerwerk (DE)**

(72) Erfinder: **Schündehütte, Karl Heinz, Dr.**
**Klief 75**
**D-5090 Leverkusen 3 (DE)**
Erfinder: **Klauke, Erich, Dr.**
**Eichendorffweg 8**
**D-5068 Odenthal (DE)**

**Die Akte enthält technische Angaben, die nach dem Eingang der Anmeldung eingereicht wurden und die nicht in dieser Patentschrift enthalten sind.**

**EP 0 019 785 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind Reaktivfarbstoffe der Formel

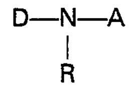 (I)

worin

R = H, Alkyl, insbesondere $C_1$—$C_4$-Alkyl

A = 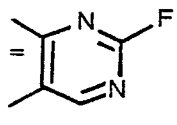

D = Rest eines Farbstoffs der Formeln

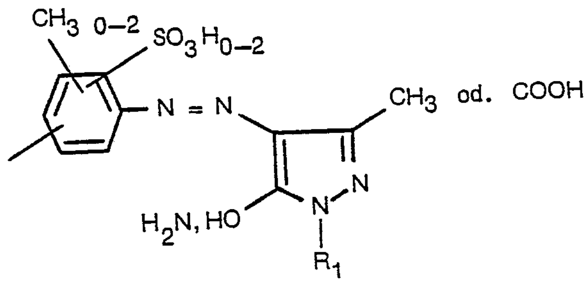

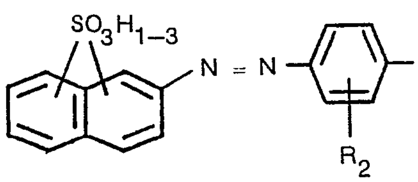

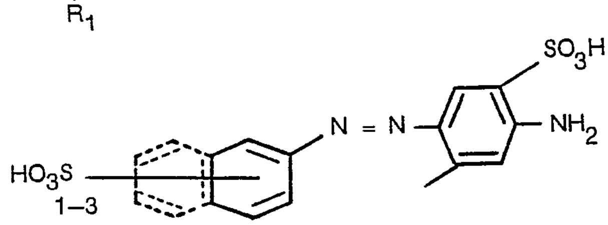

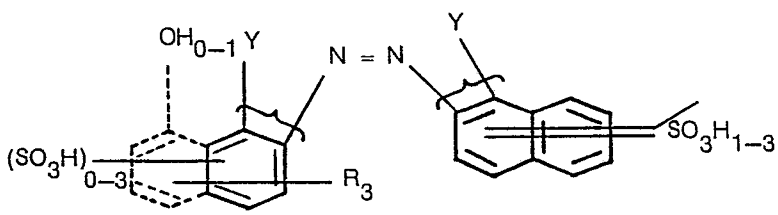

(Metallkomplexe) ,

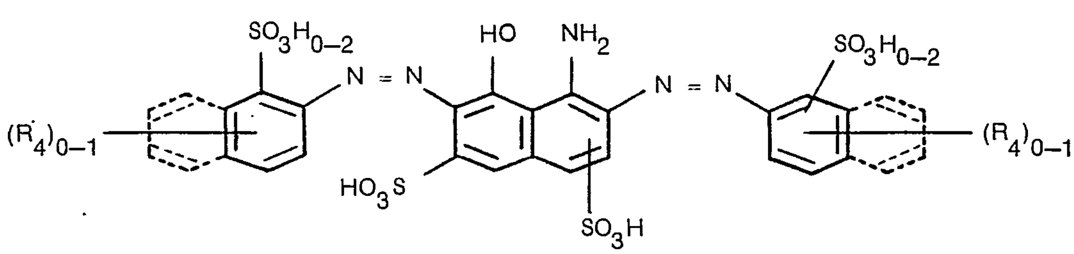

oder

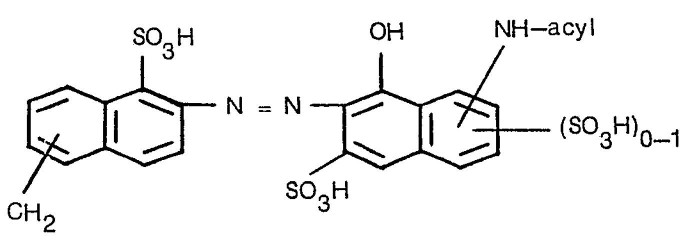

worin

$R_1$ = H, Alkyl, Aryl

$R_2$ = H, Alkyl, Alkoxy, Arylamino

$R_3$ = H, Halogen, $NO_2$, Alkyl

$R_4$ =

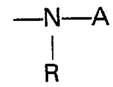

bedeutet, wobei das Molekül nur eine Gruppe $R_4$ aufweisen soll, acyl = Acylrest, insbesondere Formyl, $C_1$—$C_4$-Alkylcarbonyl, Arylcarbonyl, speziell gegebenenfalls substituiertes Phenylcarbonyl, y = metallkomplexbildende Gruppe, insbesondere — $O^{\ominus}$ oder —$COO^{\ominus}$.

Die Alkyl-, Aryl- und Alkoxyreste in den obenstehenden Formeln können gegebenenfalls weitere übliche Substituenten aufweisen. Bevorzugt sind im allgemeinen die nichtsubstituierten Reste, sowie im Rahmen der Alkyl- und Alkoxyreste solche mit 1—4 C-Atomen.

In der obenstehend zuletzt aufgeführten Formel steht die —$CH_2$-Gruppe vorzugsweise in 5-Stellung.

Aus US—A 3 822 263 sind ähnlich strukturierte Reaktivfarbstoffe bekannt, die jedoch gegenüber den erfindungsgemäßen Farbstoffen unterlegene färberische Eigenschaften aufweisen.

Die Farbstoffe dieser Formeln eignen sich in hervorragender Weise zum Bedrucken und Färben von textilen Gebilden aus nativer und regenerierter Cellulose sowie von natürlichen oder synthetischen Polypeptiden.

Die mit diesen Farbstoffen in hohen Ausbeuten erhältlichen Färbungen zeichnen sich durch eine hohe Faser-Farbstoff-Bindungsstabilität aus sowie durch eine hervorragende Stabilität gegenüber Oxydationsmitteln wie peroxyd- oder chlorhaltige Waschmittel. Die Auswaschbarkeit der bei Färben oder Drucken entstehenden Hydrolyseprodukte ist ausgezeichnet.

Die neuen Farbstoffe der allgemeinen Formel I sind nach den bei der Synthese von Reaktivfarbstoffen üblichen Herstellungsverfahren zugänglich. So können beispielsweise die entsprechenden Farbstoffe der Formel

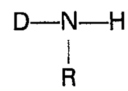

die aus der Literatur in großer Zahl bekannt sind mit 1 Mol 2,4-Difluor-5-chlor-pyrimidin kondensiert werden. Häufig wird auch die Kondensation des Fluorpyrimidins mit einem Farbstoffvor- oder -zwischenprodukt zuerst durchgeführt, der dann der Aufbau des Farbstoff z.B. durch die Azo-Kupplung folgt.

Die Umsetzung mit dem Pyrimidin erfolgt in wässriger oder organisch-wässrigen Medien bei 20—70° in Anwesenheit von säurebindenden Mitteln wie Natriumcarbonat, Natriumbicarbonat oder verdünnter Natronlauge.

Andere Umwandlungsreaktionen der Farbstoffe oder deren Vorprodukte wie Metallisierungsreaktionen, Sulfierungen oder Einführung von Acylaminogruppierungen können im allgemeinen in beliebigen Stufen der Farbstoffsynthesen vorgenommen werden.

Die Herstellung der als Ausgangsmaterial eingesetzten Reaktivkomponente 2,4-Difluor-5-chlorpyrimidin erfolgt durch Cl/F-Austausch an 2,4,5-Trichlorpyrimidin auf zweierlei Weise.

1) Mit NaF,

a) ohne Lösungsmittel in 5—15 h bei etwa 300—350°C

b) im Lösungsmittel, bspw. Tetramethylensulfon in 5—10 h bei etwa 160—230°C

2) Mit HF wasserfrei bei 80—160°C und einem Druck von 15 bis 30 bar. Dabei wird der durch die Reaktion freiwerdende Chlorwasserstoff kontinuierlich in der Weise entspannt, daß der Druck anfänglich wenig (etwa bis zu 5 bar) über und gegen Ende der Reaktion nahezu oder vollständig beim Partialdruck der wasserfreien HF liegt. Verfahren und physikalische Eigenschaften sind im Beispiel 1 näher beschrieben.

Beispiel 1

In einem V4A-Stahlautoklaven mit Rückflußkühler, Rührer und einem Entspannungsventil werden 800 ml wasserfreie HF vorgelegt und bei einer Temperatur von 0—10°C 367 g 2,4,5-Trichlorpyrimidin (Kp: 96°/12 mm, $n_D$20:1.5734) zugetropft. Der Autoklav wird verschlossen, 8 bar $N_2$ aufgedrückt und anschließend unter Rühren hochgeheizt. Die Cl/F-Austauschreaktion setzt bei 80—90°C ein, was über den Druckanstieg zu beobachten ist. Bei einem Entspannungsdruck von 20 bar und einer Temperatur von etwa 100°C läßt man die jetzt zügige HCl-Entwicklung ablaufen und heizt dann im Maße dieser HCl-Entwicklung in etwa zwei Stunden auf 135°C. Der Entspannungsdruck wird dabei auf 20 bar gehalten. 1 1/2 h bei 135—140°C nachrühren. Die HCl-Entwicklung ist danach fast beendet. Nun wird in 90 Min. der Druck von 20 bar bis auf 17 bar entspannt. Die Innentemperatur fällt dabei bis

133°C. Der Ansatz wird abgekühlt und durch fraktionierte Destillation aufgearbeitet. An Reinfraktionen werden erhalten: 206 g 2,4-Difluor-5-chlorpyrimidin Kp: 132—4°C, $n_D$20:1.4630 und 43 g 4-Fluor-2,5-dichlorpyrimidin Kp: 179—81°C, $n_D$20:1.5191.

Beispiel 2

19 Teile 1,3-Diaminobenzol-6-sulfonsäure werden in 250 Teilen Wasser angerührt und durch Zugabe von ca. 40 ml einer 20%igen Natriumcarbonatlösung gelöst.

In diese Lösung werden bei ca. 30° 15 Teile 2,4-Difluor-5-chlorpyrimidin gegeben und bei 20—50° unter kräftigem Rühren solange die freiwerdende Flußsäure durch Zutropfen einer Natriumcarbonatlösung neutralisiert bis sich der pH-Wert von 6—7 nicht mehr ändert. Das so erhaltene Kondensationsprodukt der Formel

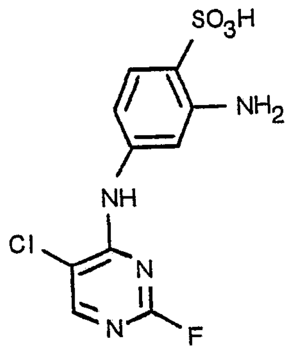

wird nach Zugabe von Eis und 7 Teilen Natriumnitrit mit 28 Teilen Salzsäure direkt diazotiert.

In diese Suspension der Diazoniumverbindung werden 30 Gewichtsteile 1-[2'-Methyl-4'-sulfophenyl]-3-carboxypyrazolon-(5) gegeben und die Kupplung durch Zugabe von ca. 80 Teile einer Natriumcarbonatlösung (20%ig) zu Ende geführt. Nach beendeter Kupplung wird die Abscheidung des Farbstoffs durch Zugabe von 200 Teilen Kaliumchlorid oder Natriumchlorid vervollständigt, anschließend wird durch Filtration isoliert und bei 50—100° getrocknet. Man erhält ein gelbes Pulver, das Baumwolle in grünstichig gelben Tönen anfärbt bzw. druckt.

Die so erhaltenen Drucke oder Färbungen bestizen hervorragende Naßechtheiten.

Ersetzt man in diesem Beispiel die Kupplungskomponente 1-(2'-Methyl-4'-sulfophenyl)-3-carboxypyrazolon-(5) durch äquimolare Mengen der in der folgenden Tabelle aufgeführten Pyrazole, so werden ebenfalls gelbe Reaktivfarbstoffe erhalten.

| Bsp. | Acyliertes Diamin | Kupplungskomponente |
|---|---|---|
| 3 | 1,3-Diaminobenzol-6-sulfonsäure | 3-Methylpyrazolon-(5) |
| 4 | 1,3-Diaminobenzol-6-sulfonsäure | 1,3-Dimethylpyrazolon-(5) |
| 5 | 1,3-Diaminobenzol-6-sulfonsäure | 1-Carboxymethyl-3-methylpyrazolon-(5) |
| 6 | 1,3-Diaminobenzol-6-sulfonsäure | 1-(4'-Sulfophenyl)-3-carboxypyrazolon-(5) |
| 7 | 1,3-Diaminobenzol-6-sulfonsäure | 1-(2'-5'-Disulfophenyl)-3-methylpyrazolon-(5) |
| 8 | 1,3-Diaminobenzol-6-sulfonsäure | 1-(2'-Chlor-5'-sulfophenyl-3-methylpyrazolon-(5) |
| 9 | 1,3-Diaminobenzol-6-sulfonsäure | 1-(3'-Sulfophenyl)-3-methyl-5-aminopyrazol |

Beispiel 10

Die Lösung von 21 Teilen 1,3-Diamino-2-methylbenzol-5-sulfonsäure als Natriumsalz in 300 Teilen Wasser wird bei 20—50° unter kräftigem Rühren mit 15 Teilen 2,4-Difluor-5-chlorpyrimidin kondensiert. Die freiwerdende Flußsäure wird dabei durch Zutropfen einer 20%igen Sodalösung neutralisiert. Nach beendeter Kondensation wird das Umsetzungsprodukt durch Zugabe von Eis, 7 Teilen Natriumnitrit und 28 Teilen Salzsäure bei 5—10° direkt diazotiert und anschließend durch Zugabe von 30 Gewichtsteilen 1-(2'-Methyl-4'-sulfophenyl)-3-carboxypyrazolon-(5) und ca. 80 Teilen einer 20%igen Sodalösung zum Azofarbstoff der Formel

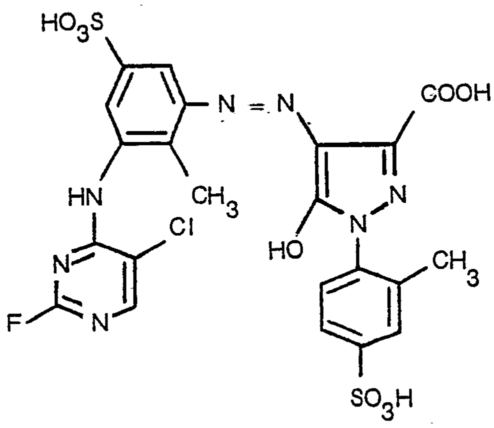

gekuppelt. Die Abscheidung des Farbstoffs wird durch Aussalzen mit 150 Teilen Natriumchlorid vervollständigt. Anschließend wird durch Filtration isoliert und bei 50—100° getrocknet. Man erhält ein gelbes Pulver, das zum Bedrucken und Färben von Baumwolle hervorragend geeignet ist.

Ersetzt man in diesem Beispiel die Kupplungskomponente durch die in den Beispielen 3—9 angegebenen Pyrazolderivate, so werden ebenfalls gelbe Reaktivfarbstoffe mit sehr guten färberischen Eigenschaften erhalten.

Beispiel 11

19 Teile 1,4-Diaminobenzol-2-sulfonsäure werden als Natriumsalz in 250 Teilen Wasser bei 20° gelöst und mit 15 Teilen 2,4-Difluor-5-chlor-pyrimidin bei dieser Temperatur selektiv kondensiert. Durch Zugabe von 30 Teilen einer 20%igen Sodalösung wird dabei ein pH-Wert von 5,8—6 eingehalten.

Nach beendeter Kondensation wird die Suspension durch Zagabe von Eis, 7 Teilen Natriumnitrit und 28 Teilen Salzsäure direkt diazotiert.

Die Suspension der Diazoniumverbindung wird mit einer Lösung des Natriumsalzes von 29 Teilen 1-(4'-Sulfophenyl)-3-carboxypyrazolon-(5) in 150 Teilen Wasser versetzt und die Kupplung durch Zugabe von 50 Teilen einer 20%igen Sodalösung zu Ende geführt. Danach wird mit 200 Teilen Kaliumchlorid ausgesalzen, isoliert und getrocknet. Man erhält so ein gelbes Pulver, das Baumwolle in goldgelben Farbtönen färbt. Der Farbstoff entspricht der Formel:

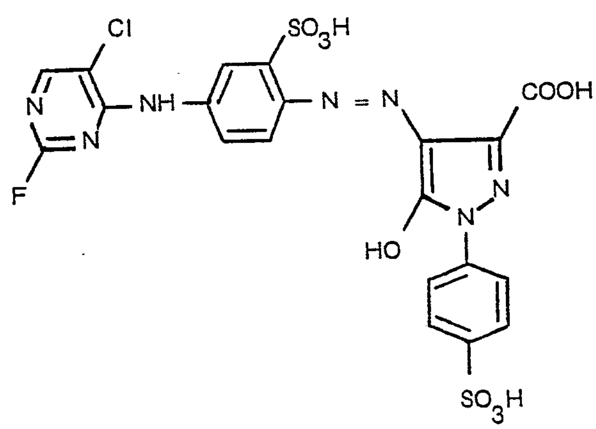

Ähnliche Farbtöne werden erhalten, wenn in diesem Beispiel das 1-(4'-Sulfophenyl)-3-carboxypyrazolon-(5) durch äquimolare Mengen der in Beispiel 3—9 angegebenen Pyrazole ersetzt wird.

Beispiel 12

Zur Lösung von 0,1 Mol des nach üblichen Methoden hergestellten Aminoazofarbstoffs der Formel

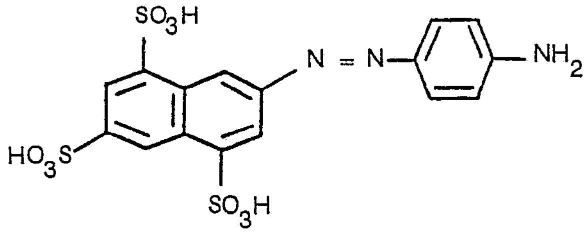

in 1000 Teilen Wasser werden bei pH 7 und ca. 50° 15 Teile 2,4-Difluor-5-chlor-pyrimidin zugetropft und dabei der pH-Wert durch Zugabe von verdünnter Natronlauge oder einer Natriumcarbonatlösung im Bereich von 6—8 gehalten.

Nach beendeter Kondensation wird ausgesalzen, isoliert und getrocknet. Der als Gelbes Pulver anfallende Farbstoff der Formel

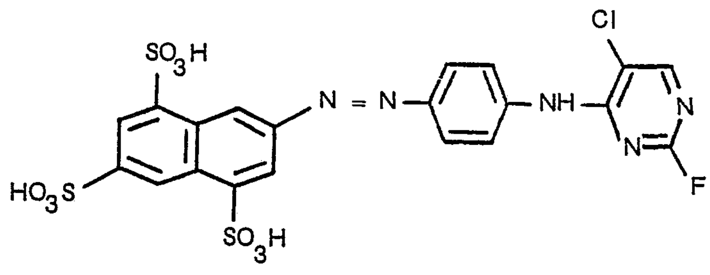

färbt Baumwolle in licht- und chlorechten gelben Farbtönen.

Wird in diesem Beispiel die 2-Aminonaphthalin-4,6,8-trisulfonsäure durch 2-Aminonaphthalin-4,8-disulfonsäure, 2-Aminonaphthalin-6,8-disulfonsäure, 2-Aminonaphthalin-3,6,8-trisulfonsäure, ersetzt, so werden ebenfalls gelbe Reaktivfarbstoffe hervorragender Licht- und Naßechtheiten erhalten.

In analoger Weise können gelbe Reaktivfarbstoffe dieses Typs erhalten werden, wenn in diesem Beispiel das als Kupplungskomponente eingesetzte Anilin durch 3-Methylanilin oder durch 2-Methoxyanilin ersetzt wird.

Beispiel 13

Zur Lösung der nach üblichen Methoden aus 0,1 Mol 2-Aminonaphthalin-4,6,8-trisulfonsäure zu erhaltenden Diazoniumverbindung werden 0,1 Mol des nach Beispiel 2 zu erhaltenden Kondensationsproduktes aus 19 Teilen 1,3-Diaminobenzol-6-sulfonsäure und 15 Teilen 2,4-Difluor-5-chlor-pyrimidins gegeben und die Kupplung durch Neutralisation mit einer verdünnten Sodalösung zu Ende geführt.

Nach Aussalzen, Filtrieren und Trocknen wird der Farbstoff der Formel

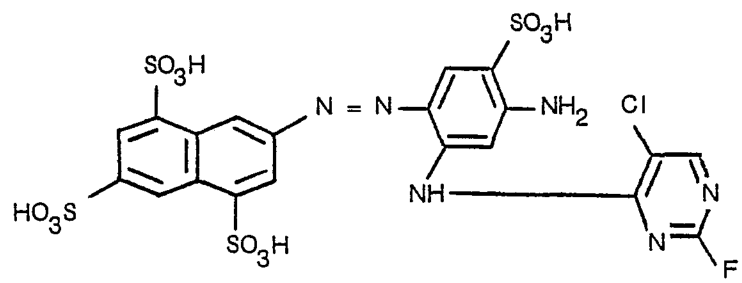

in Form eines gelben Pulvers erhalten. Er eignet sich in hervorragender Weise zum Färben und Bedrucken von Baumwolle in goldgelben Farbtönen.

Beispiel 14

In die Lösung des Natriumsalzes von 32 Gewichtsteilen 1-Amino-8-hydroxynaphthalin-3,6-disulfonsäure in 200 Teilen Wasser werden bei pH 5,5—6 wie in Beispiel 15 angegeben 15 Teile 2,4-Difluor-5-chlor-pyrimidin gegeben und bei 30—40° kondensiert.

Die Lösung des Kondensationsproduktes wird dann mit der Diazoniumverbindung aus 0,1 Mol 2-Aminonaphthalin-4,6,8-trisulfonsäure in 200 Teilen Wasser vereinigt und durch Zugabe von Alkali die Kupplung bei pH 7 durchgeführt.

Nach beendeter Kupplung wird die rote Farbstofflösung mit 35 Gewichtsteilen Kupfersulfat versetzt und bei 15° und pH 4,6 eine Mischung aus 50 Teilen einer 30%igen Wasserstoffperoxydlösung und 130 Teilen einer 20%igen Natriumacetatlösung zugetropft. Durch Zugabe von ca. 200 g Eis wird dabei die Temperatur im Bereich von 10—15° gehalten. Nach beendeter oxydativer Kupferung wird der Kupferkomplexfarbstoff der Formel

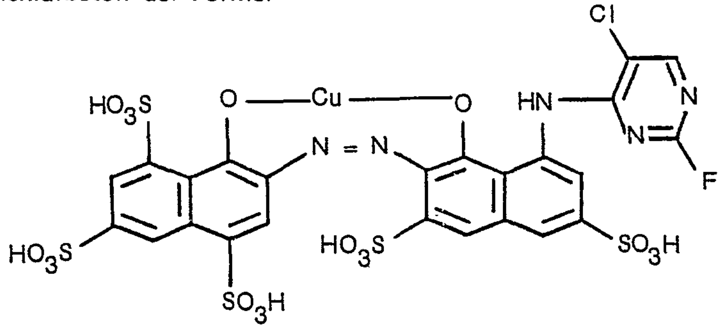

ausgesalzen, isoliert und getrocknet. Man erhält ein dunkles Pulver, das sich in Wasser mit blauer Farbe löst und zum Bedrucken und Färben von Baumwolle in marineblauen Farbtönen mit guter Lichtechtheit geeignet ist.

Die Diazoniumverbindung aus 2-Naphthylamin-4,6,8-trisulfonsäure kann in diesem Beispiel durch die Diazoniumverbindungen aus den Aminen der folgenden Tabelle ersetzt werden. Die Ausführung der oxydativen Kupferung erfolgt analog.

| Diazoniumverbindung | Farbton |
|---|---|
| Anilin-4-sulfonsäure | Violett |
| Anilin-3,5-disulfonsäure | Violett |
| Anilin | Rotviolett |
| 2-Aminonaphthalin-4,8-disulfonsäure | Blau |
| 2-Aminonaphthalin-6,8-disulfonsäure | Blau |
| 2-Aminonaphthalin-1,5,7-trisulfonsäure | Blau |

Beispiel 15

In die nach dem in Beispiel 14 beschriebenen Verfahren zur Herstellung des Kondensationsproduktes aus 2-Amino-5-hydroxynaphthalin-7-sulfonsäure und 2,4-Difluor-5-chlor-pyrimidin erhaltene Lösung wird eine Suspension der Diazoniumverbindung aus 0,1 Mol 2-Aminonaphthalin-4,6,8-trisulfonsäure in 250 Volumenteilen Wasser gegeben und die Kupplung durch Zugabe von 20 Teilen Natriumbikarbonat zu Ende geführt. Die so erhaltene Kupplungslösung wird mit 250 Teilen einer 20%igen Natriumacetatlösung und 500 Teilen einer 3%igen Wasserstoffperoxydlösung versetzt. Die oxydative Kupferung wird dann durch Zutropfen von 250 Teilen einer 20%igen Kupfersulfatlösung erreicht. Nach beendeter Kupferung wird der Farbstoff der Formel

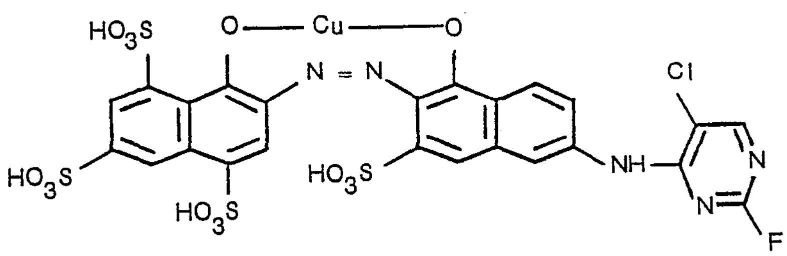

mit einem Gemisch aus 400 Teilen Natriumchlorid und 100 Teilen Kaliumchlorid ausgesalzen anschließend isoliert und getrocknet. Man erhält ein dunkles Pulver, das sich in Wasser mit violetter Farbe löst und Baumwolle in klaren blauvioletten Farbtönen färbt.

Ersetzt man in diesem Beispiel die Diazoniumverbindung aus 2-Aminonaphthalin-4,6,8-trisulfonsäure durch Diazoniumverbindungen aus folgenden aromatischen Aminen, so erhält man ebenfalls violette Reaktivfarbstoffe:

Anilin-4-sulfonsäure
Anilin-3-sulfonsäure
Anilin-3,5-disulfonsäure
2-Aminonaphthalin-4,8-disulfonsäure
2-Aminonaphthalin-6,8-disulfonsäure
1-Aminonaphthalin-4,6-disulfonsäure
1-Aminonaphthalin-4,7-disulfonsäure
2-Aminonaphthalin-3,6,8-trisulfonsäure.

Beispiel 16

0,1 Mol der Aminoazoverbindung der Formel

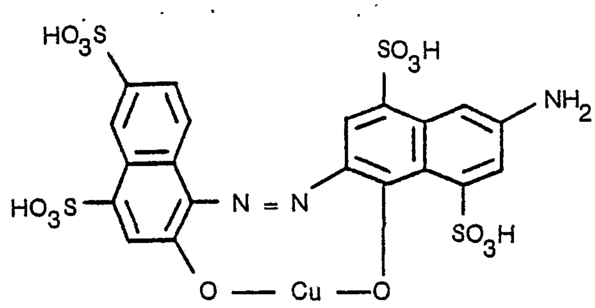

die durch Kupplung von 1-Aminonaphthalin-4,6-disulfonsäure mit 2-Acetylamino-5-hydroxynaphthalin-4,8-disulfonsäure anschließender oxydativer Kupferung und Verseifung der Acetylaminogruppe erhalten werden kann, werden in 1500 Volumenteilen Wasser bie pH 6,5 gelöst und bei ca. 50° mit 16 Teilen 2,4-Difluor-5-chlorpyrimidin portionsweise versetzt, wobei die freiwerdende Säure so mit einer Sodalösung neutralisiert wird, daß während der Kondensationszeit von etwa 2 Stunden ein pH von 6,5 gehalten wird.

Nach beendeter Umsetzung wird der Farbstoff der Formel

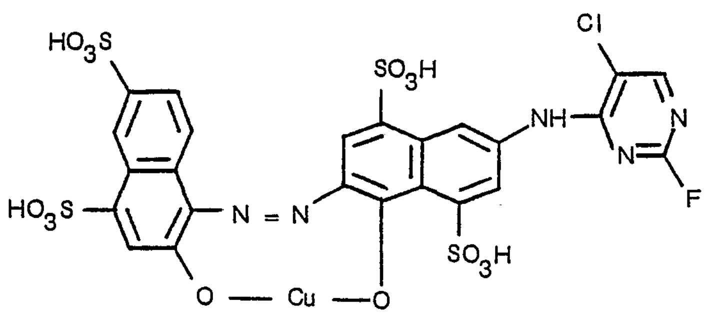

ausgesalzen, isoliert und bei ca. 50° im Vakuum getrocknet.

Man erhält ein dunkles Pulver, das sich in Wasser mit blauer Farbe löst und Baumwolle in rotstichig blauen Farbtönen färbt. Der gleiche Farbstoff kann durch Kupplung der Diazoniumverbindung aus 1-Aminonaphthalin-4,6-disulfonsäure mit dem Kondensationsprodukt aus 2-Amino-5-hydroxynaphthalin-4,8-disulfonsäure und 2,4-Difluor-5-chlor-pyrimidin und anschließender oxydativer Kupferung erhalten werden.

Farbstoffe, die Baumwolle in ähnlichen blauen Farbtönen färben, können erhalten werden, wenn man in diesem Beispiel die Diazoniumverbindung aus 1-Aminonaphthalin-4,6-disulfonsäure durch die der folgenden Amine ersetzt:

1-Aminonaphthalin-4,7-disulfonsäure
1-Aminonaphthalin-4-sulfonsäure
2-Aminonaphthalin-6,8-disulfonsäure
2-Aminonaphthalin-4,6,8-trisulfonsäure
2-Aminonaphthalin-3,6-disulfonsäure
1-Amino-8-hydroxynaphthalin-4,6-disulfonsäure bzw. -3,6-disulfonsäure.

Beispiel 17

0,1 Mol des Kondensationsproduktes aus 1,3-Diaminobenzol-6-sulfonsäure und 2,4-Difluor-5-chlor-pyrimidin wird wie in Beispiel 2 beschrieben diazotiert und die Suspension bei pH 2—3 mit 0,1 Mol 1-Amino-8-hydroxynaphthalin-3,6-disulfonsäure gekuppelt. Nach beendeter Kupplung wird die dunkelrote Farbstofflösung mit der Suspension der Diazotierung aus Anilin-2,5-disulfonsäure versetzt. Zur zweiten Kupplung wird der pH-Wert des Reaktionsgemisches durch Zutropfen einer 20%igen Sodalösung auf 7—8 gestellt. Nach beendeter Kupplung wird der Farbstoff der Formel

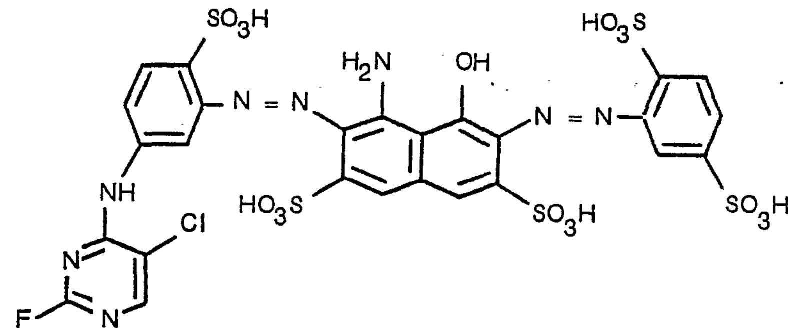

ausgesalzen, isoliert und getrocknet. Erstellt dann ein dunkles Pulver dar, das sich in Wasser mit blauer Farbe löst und Baumwolle in dunkelblauen bis schwarzen Farbtönen färbt.

Beispiel 18

Die in schwach saurem pH-Bereich ausgeführte Kupplung von 0,1 Mol der Diazoniumverbindung aus Anilin-2,5-disulfonsäure mit 0,1 Mol 1-Amino-8-hydroxynaphthalin-3,6-disulfonsäure führt zu einer dunkelroten Lösung des Monoazofarbstoffs, der bei pH 6—7 mit 0,1 Mol der Diazoniumverbindung aus dem Kondensationsprodukt von 1,3-Diaminobenzol-6-sulfonsäure und 2,4-Difluor-5-chlor-pyrimidin zu einem dunkelblauen Farbstoff gekuppelt werden kann. Nach Aussalzen, Isolieren und Trocknen fällt der Farbstoff der Formel

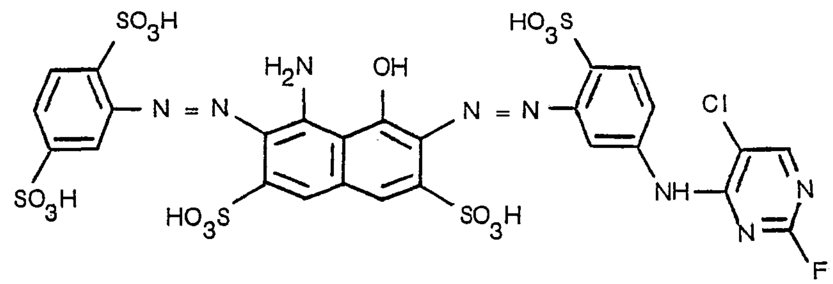

als dunkles Pulver an, das sich in Wasser mit blauer Farbe löst und Baumwolle in dunkelblauen bis schwarzen Farbtönen färbt.

**Patentansprüche**

1. Reaktivfarbstoffe der Formel

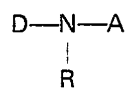 (I)

worin

R = H, Alkyl, insbesondere $C_1$—$C_4$-Alkyl

A = 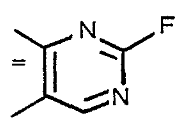

D = Rest eines Farbstoffs der Formeln

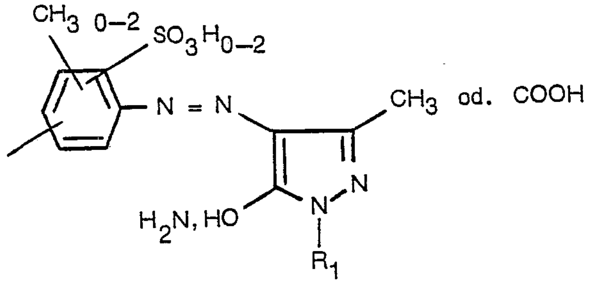

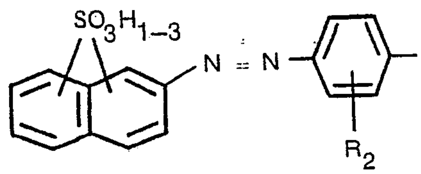

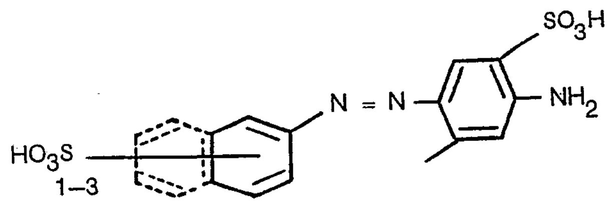

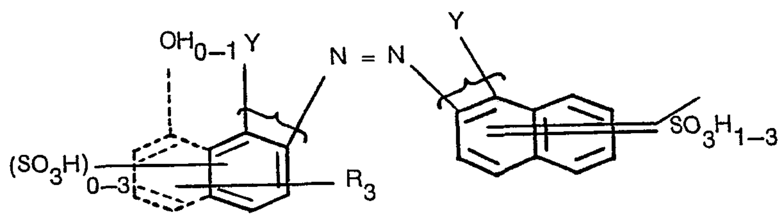

(Metallkomplexe) ,

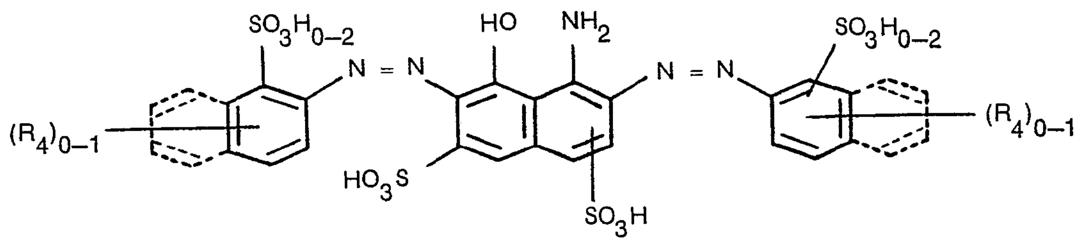

oder

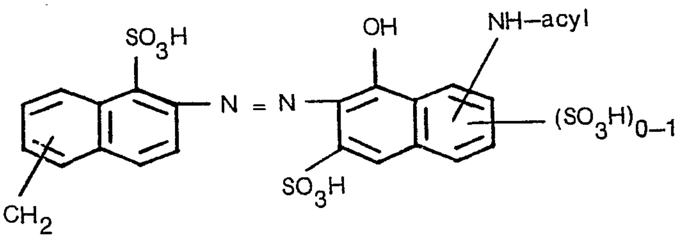

worin

$R_1$ = H, Alkyl, Aryl
$R_2$ = H, Alkyl, Alkoxy, Arylamino
$R_3$ = H, Halogen, $NO_2$, Alkyl
$R_4$ =

$$\begin{array}{c}-N-A\\ |\\ R\end{array}$$

bedeutet, wobei das Molekül nur eine Gruppe $R_4$ aufweisen soll,
acyl = Acylrest, insbesondere Formyl, $C_1$—$C_4$-Alkylcarbonyl, Arylcarbonyl, speziell gegebenenfalls substituiertes Phenylcarbonyl, y = metallkomplexbildende Gruppe, insbesondere — $O^{\ominus}$ oder —$COO^{\ominus}$.

2. Verwendung der Farbstoffe des Anspruch 1 zum Färben und Bedrucken von Cellulose oder Polypeptiden.

**Revendications**

1. Colorants réactifs de formule

$$\begin{array}{c}D-N-A\\ |\\ R\end{array} \qquad (I)$$

dans laquelle

R = H, alkyle, en particulier alkyle en $C_1$—$C_4$,

A = 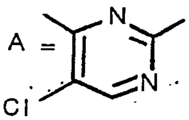

D = reste d'un colorant répondant à l'une des formules

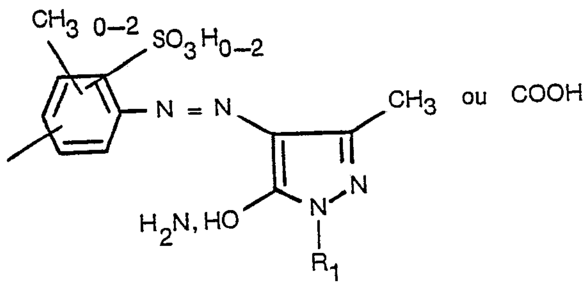

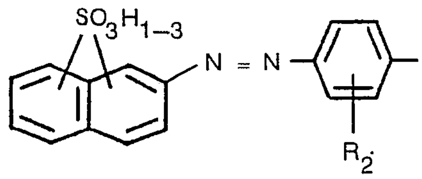

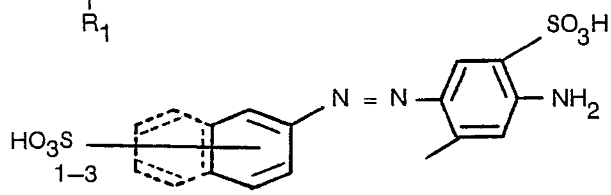

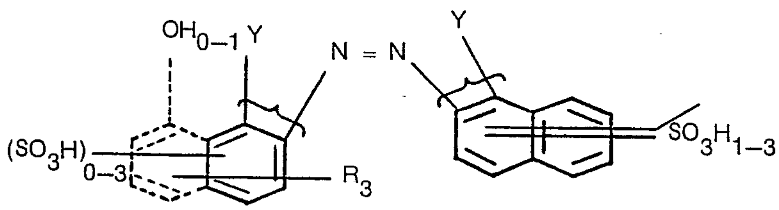

(complexes métalliques),

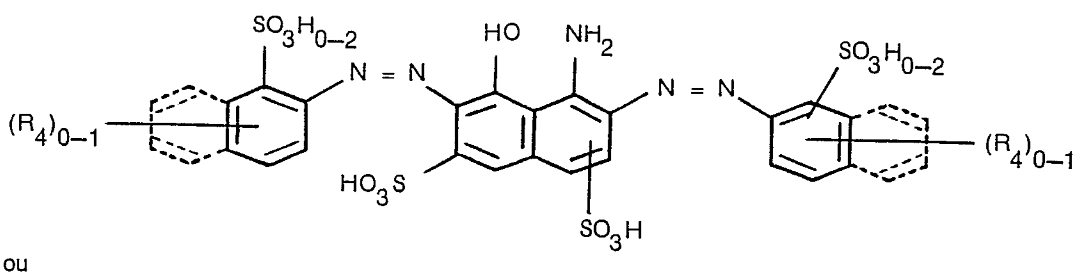

ou

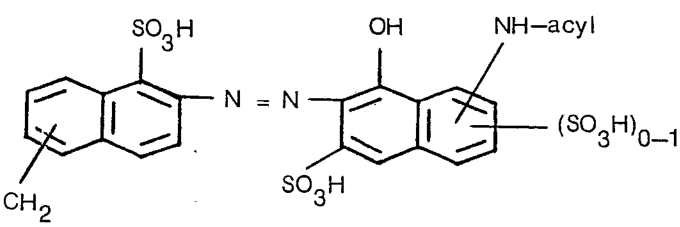

dans lesquelles

$R_1$ = H, alkyle, aryle
$R_2$ = H, alkyle, alcoxy, arylamino
$R_3$ = H, halogène, $NO_2$, alkyle
$R_4$ =

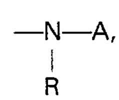

la molécule ne devant porter qu'un seul groupe $R_4$,
"acyle" = radical acyle, en particulier formyle, (alkyle en $C_1$—$C_4$)-carbonyle, arylcarbonyle, spécialement phénylcarbonyle éventuellement substitué, y = groupe formant des complexes métalliques, en particulier culier —$O^\ominus$ ou —$COO^\ominus$.

2. Utilisation des colorants de la revendication 1 pour la teinture et l'impression de la cellulose ou des polypeptides.

**Claims**

1. Reactive dyestuffs of the formula

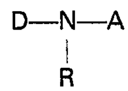 (I)

wherein

R denotes H or alkyl, in particular $C_1$—$C_4$-alkyl,

A denotes

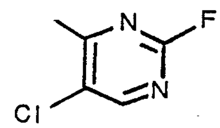

D denotes the radical of a dyestuff of the formulae

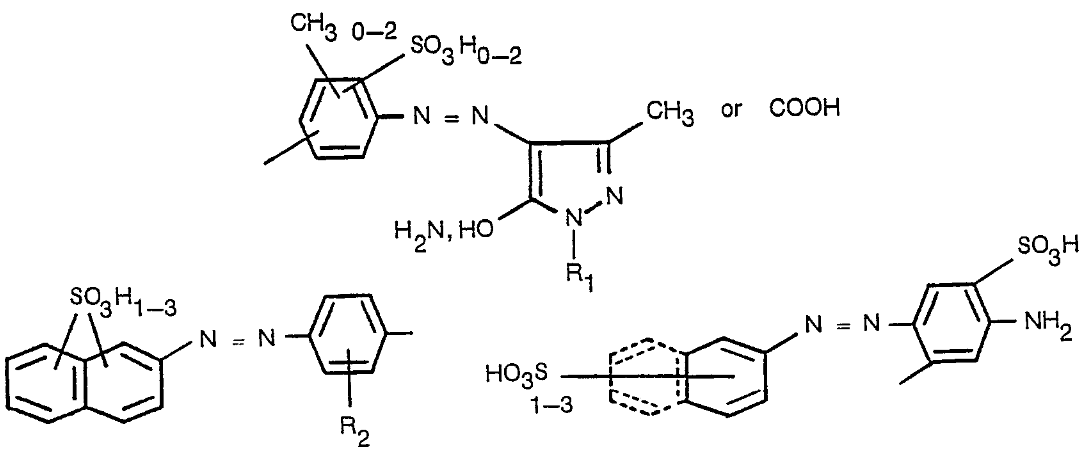

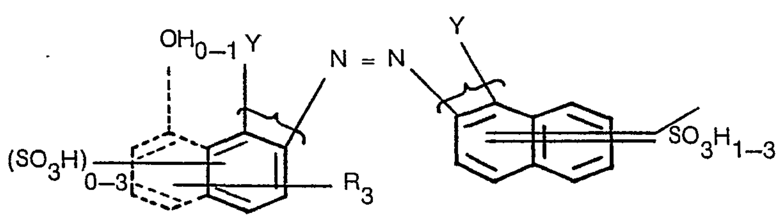

(metal complexes),

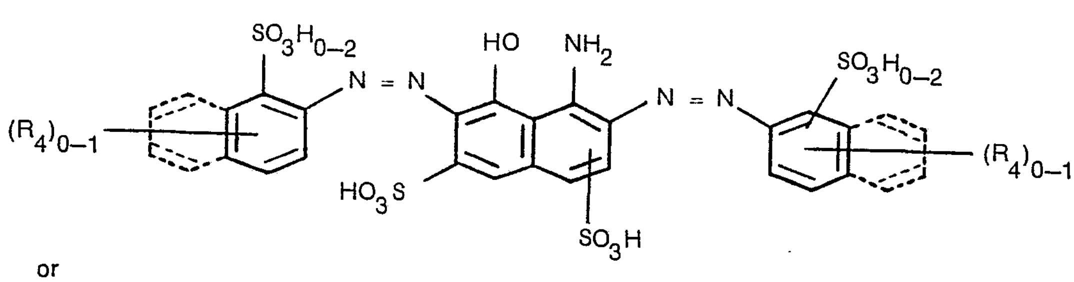

or

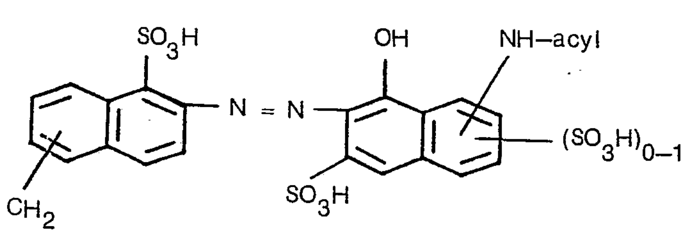

wherein

$R_1$ denotes H, alkyl, or aryl,

$R_2$ denotes H, alkyl, alkoxy or arylamino,

$R_3$ denotes H, halogen, $NO_2$ or alkyl,

$R_4$ denotes

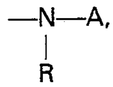

but the molecule should contain only one group $R_4$,

acyl = an acyl radical, in particular formyl, $C_1$—$C_4$-alkylcarbonyl or arylcarbonyl, in particular optionally substituted phenylcarbonyl, and y = a metal complex-forming group, in particular —$O^{\ominus}$ or —$COO^{\ominus}$.

2. Use of the dyestuffs of Claim 1 for dyeing and printing cellulose or polypeptides.